# EUROPEAN PATENT APPLICATION

(11) **EP 0 747 073 A1**
(43) Date of publication of application: **11.12.1996**
(21) Application number: 96201488.2
(22) Date of filing: 29.05.1996
(51) Int. Cl.: A61L 33/00, A61M 25/01

(54) **Medical device with reduced frictional properties and method for reducing friction**

(30) Priority: 08.06.1995 NL 1000526
(71) Applicant: CORDIS EUROPA N.V., NL-9301 LJ Roden (NL)
(72) Inventor: Bos, Johannes Jacobus Gerardus, 9977 RX Kloosterburen (NL)
(74) Representative: Van Someren, Petronella F. H. M.

(57) **Abstract**

The invention relates to a medical device with reduced frictional properties, characterized in that at least two of the contact surfaces inside the device and/or between the device and its environment are provi- ded with means for reducing, changing and/or eliminating biological/biochemical responses of the human or animal body and/or human or animal body fluids which occur in reaction to the contact with the device. The device may further comprise means for providing a fluid layer be- tween the contact surfaces inside the device and/or between the device and its environment.

## Description

The present invention relates to a medical device with reduced frictional properties and to a method for reducing friction in or through medical devices.

In the field of medical devices different types of system are being developed which are used in vivo in humans and animals or come into contact ex vivo with human or animal body fluids. The contact between the device and in particular the inner surfaces of a human or animal or their body fluids can cause different reactions. The presence of the device can thus result in biological/biochemical responses such as for instance blood clotting. Such responses of course have an adverse effect on the patient but also result in interaction between the device and the body proceeding less smoothly, whereby pain may for instance be caused or whereby clinical procedures become unnecessarily long or even impossible.

A type of device to which this particularly applies are for instance coaxial catheter systems which come into contact with blood. In such systems the one catheter moves through the other. The outer surface of the inner catheter and the inner surface of the outer catheter can herein come into mutual contact. A liquid may, intentionally or-unintentionally, be present between the two catheters such as for instance the blood itself, which can facilitate the movement but, depending on the rheological properties of the liquid, may also make the movement more difficult. The movement of the two tubes relative to each other will however result in most cases in undesirably high friction. Situations where undesired friction occurs may cause an interruption of the clinical treatment, which is unpleasant and in particular cases also adversely affects the patient.

Different methods are per se known for minimizing friction. Materials with a low coefficient of friction such as polytetrafluoroethylene (PTFE), also known under the name Teflon®, and polyethylene are already in general use.

In addition, friction can be reduced by applying lubricants, wherein the relevant surfaces are mutually separated by a fluid in the liquid or gas phase. Characteristic of the liquid and gas phase is that the molecules of the fluid can move freely relative to one another. To what extent this movement takes place depends on the viscosity of the fluid. The higher the viscosity the more difficult it becomes to cause molecules to displace relative to one another. This has the consequence that at a high viscosity more friction will occur.

Another problem which occurs when lubricants are applied between surfaces moving along each other is that too low a viscosity can result in the liquid displacing too easily and getting squeezed out between the two surfaces. This has the result that the surfaces once again come into mutual contact, which again leads to increased friction. For optimum operation of lubricants, i.e. in order to cause a low friction, there must be a balance between the viscosity and the displacement forces or energies of the lubricant used.

The occurrence of friction can generally better be reduced by using lubricants such as water and air than by means of surfaces having a low coefficient of friction such as PTFE and polyethylene. The objective is therefore surfaces which are very well able to hold a fluid layer. There are different techniques for achieving this.

Simple adhesion and cohesion forces (van der Waals' forces) can hold the fluid on the surface. As long as there are no forces causing the fluid layer to flow away this latter will remain present due to the said forces. Surfaces with a large surface tension, also referred to as hydrophilic or wettable surfaces, will per definition hold the fluid layer best.

Also used are hydrogels which preserve a liquid layer in that when pressure is exerted thereon they gradually secrete a small quantity of the liquid absorbed by the gel. The gels must therefore be saturated with a liquid before they can be used. Re-saturation will occur at any desired moment when no or minimal forces are exerted on the gel. This in fact means that the gel reabsorbs the secreted liquid. Gels are manufactured for instance by cross-linking large polymer chains during polymerization or thereafter. The resulting structures resemble sponges.

The above described methods of preventing the occurrence of friction function extremely well when non-reactive liquids are being used. The systems will however mostly fail after a determined time when chemically reactive liquids are introduced, intentionally or unintentionally, as lubricant.

In medical devices bodily fluids often serve as lubricants but have the important drawback that they are not inert and that their chemical properties can change in the course of time.

Chemically reactive liquids (and gases) can cause inter alia corrosion of the surfaces which come into contact therewith, whereby the coefficients of friction are very adversely affected. When the viscosity of lubricants increases due to internal chemical changes the performance of these agents will decrease. Due account must be taken, certainly in the human body, of changes which can occur in bodily fluids.

When a medical device is introduced in vivo into the bloodstream different processes will occur. Constituents of the blood such as proteins, cell structures (for instance blood platelets etc.) can precipitate onto the surface of the device. The viscosity of the blood will also increase through clotting processes. Reactions can further occur with the surfaces whereby the chemical and physical structure thereof changes. The clotting products are also deposited onto the surfaces of the device whereby a top layer is created which has differing frictional properties.

An attendant physician will, depending on a number of parameters, observe an increase in friction or even find that parts of the device have become stuck. The properties of the blood such as clotting time or reaction time of other chemical mechanisms, the duration of the treatment, the actual contact region between the surfaces and the deposits thereon and the normal forces acting on the surfaces will all have an effect on the operability of the device. In coaxial systems of catheters for instance, where one catheter is guided through another, the operability will depend on the tolerances between the catheters by which the blood volume situated between the catheters is determined, the curvature of the catheter tubes, the duration of the treatment and the occurrence of reactions such as clotting which are induced by the surfaces. The natural, intrinsically caused clotting time of the blood is further-a decisive factor in the success of the treatment. In addition, the adhesive power of the deposits on the surfaces can also have an influence. Blood clots for instance do not adhere well on PTFE whereby a better friction performance will be obtained compared with a surface on which blood clots will adhere very well.

It is the object of the present invention to provide a medical device with which friction between the surfaces of the device mutually and between the device and its environment can be reduced, preferably reduced to the minimum.

The present invention is based on the insight that there is a relation between the biological/biochemical responses of the body or of the body fluids in reaction to contact with a medical device, and the friction which occurs through or in this device.

The object of the invention is therefore achieved by a device which is characterized by the fact that at least two of the contact surfaces inside the device and/or between the device and its environment are provided with means for reducing, changing and/or eliminating biological/biochemical responses of the human or animal body and/or their body fluids which occur in reaction to the contact with the device. Two surfaces coming into mutual contact are preferably provided with such means.

In a preferred embodiment the device also contains means for providing a fluid layer between the contact surfaces inside the device and/or between the device and its environment.

By taking into account the possibly occurring responses and, in anticipation thereof, adapting the contact surfaces of the device such that such biological/biochemical responses are reduced, changed and/or eliminated, the occurrence of an undesired friction can be prevented. A further reduction of the friction can be obtained by also providing a practically permanent fluid layer between the contact surfaces.

In a particularly advantageous embodiment of the invention the means for reducing, changing and/or eliminating biological/biochemical responses are formed by a surface coating layer in which anti-clotting agents are included. Particularly in the case blood is used as lubricant, anti-clotting agents will prevent the viscosity of the blood changing, whereby the lubricating properties of the blood remain optimal. Also prevented is the deposition of fibrin threads on the surfaces which could have resulted in a deterioration of the frictional properties of these surfaces. The fibrin threads can now not be formed due to the counteraction of the anti-clotting agent.

An example of an anti-clotting agent which can be used is heparin or substances derived therefrom.

The means for providing a fluid layer are preferably formed by a hydrogel.

A device which benefits particularly from the insights according to the invention is a coaxial catheter system. The outer surfaces of the inner catheter and the inner surfaces of the outer catheter are preferably provided with anti-clotting agents and optionally a hydrogel. The outer surface of the outer catheter can also be provided with such means to prevent friction of the catheter in relation to the blood vessel. In cases where a guide wire is trained through the inner catheter, the provision of a layer of anti-clotting agents and an optional fluid layer on the inner wall of the inner catheter also has certain advantages.

The invention further provides a method for reducing the frictional properties of a medical device by providing at least two of the contact surfaces inside the device and/or between the device and its environment with means for reducing, changing and/or eliminating biological/biochemical responses of the human or animal body and/or human or animal body fluids which occur in reaction to the contact with the device. Means can optionally be applied for providing a fluid layer between the contact surfaces inside the device and/or between the device and its environment.

This entails in practice that the desired means, such as anti-clotting agents and optionally also a hydrogel, are applied directly during production of the device or immediately prior to use thereof.

Understood in this application by "biological/biochemical responses" are inter alia the changes in the viscosity of body fluids located between the contact surfaces (friction surfaces), the deposition of substances from the body onto the contact surfaces and/or the chemical and/or physical change in form of the surfaces due to the action of body fluids. Other responses can of course also be referred to here. The above stated are however the most frequently occurring.

The present invention will be elucidated further with reference to the accompanying examples, which are only given by way of illustration and are not intended to limit the invention in any way. Example 1 is a comparison example-indicating the effects which can occur in the body during use of a catheter. Examples 2 and 3 give embodiments of the device according to the invention.

### COMPARATIVE EXAMPLE 1

From animal experiments and from clinical practice it has been found that the chance of guide wires coated with PTFE and/or silicone grease becoming stuck in a catheter introduced into a living body increases as the dimensions of both devices decrease. Typical diameters are 0.4 mm for the guide wire and 0.7 mm for the catheter's. This type of catheter is common used in angioplasty treatments which require more time than diagnostic procedures, whereby there are longer contact times between the blood and the surfaces of the guide wire and the catheter. It was always found in vitro making use of non-reactive liquids instead of blood that the guide wire could move freely through the catheter.

### EXAMPLE 2

During PTCA (percutaneous transluminal coronary angioplasty) treatments, also referred to as balloon or dotter treatments, a balloon catheter is introduced into the body by a guiding catheter already positioned close to the place of the intended intervention. In this experiment a dog had a guide catheter introduced of a nylon-polymer blend with an inner diameter of 1½ mm, an outer diameter of 2.0 mm and a length of 1 metre. The inner and outer surfaces of the catheter were coated with heparin. The balloon catheter was likewise made from a nylon blend and was tested in two configurations, uncoated and coated with heparin. At the site of the balloon the balloon catheter had an outer diameter between 0.8 and 1.2 mm. The outer diameter of the remaining parts of the catheter amounted to 0.8 mm, while the length was 135 cm.

It was found that the uncoated balloon catheter after 5 to 10 minutes of continuous movement became completely stuck in the guiding catheter. The balloon catheter with the anti-thrombogenic coating could in contrast be moved freely and easily for a period of more than 10 minutes. In both cases the guiding catheter was filled with blood prior to insertion of the balloon catheter.

This experiment illustrates that preferably all surfaces involved in the friction problem must be treated in order to prevent adverse biological/biochemical reactions of, in this case, the blood. In this experiment examination showed that the surface of the non-coated balloon catheter had induced blood clotting and the coated balloon had not.

### EXAMPLE 3

In this example a coaxial catheter system was optimized by reducing adverse biochemical responses of the blood to the system and by making use of the concept that a liquid film between the two catheters must be preserved to ensure so-called "aquaplaning", and therewith permanent lubrication.

Three types of mini-catheter of polyurethane were used with an inner diameter of 0.5 mm and an outer diameter of 0.8 mm. In the first type neither the inner nor outer surfaces were coated. In the second type the inner and outer surfaces were coated with a covalently bonded anti-thrombogenic agent (heparin). In the third type the inner surface was coated with a covalently bonded anti-thrombogenic agent, while the outer surface was coated with an anti-thrombogenic hydrogel. A polyurethane tube with an inner diameter larger than or equal to 1.0 mm and an outer diameter of 1.7 mm was used as guiding catheter. Both the inner and outer surface of the guiding catheter were coated with a covalently bonded anti-thrombogenic agent. The mini-catheter can be used to reach into the smaller blood vessels.

The guiding catheter was introduced into a pig as closely as possible to the location of intervention or diagnosis, whereafter the mini-catheter was inserted. The function of the guiding catheter is to enable multiple insertion with different types of mini-catheters, including balloon catheters. After positioning of the guiding catheter the non-coated mini-catheter was inserted. The mini-catheter became stuck immediately after insertion. Its distal extremity could not even reach the distal end of the guiding catheter.

It is noted that during insertion blood will always be admitted into the guiding catheter to prevent air penetrating into the body. This explains why in this example the non-coated mini-catheter entered into a negative biological/biochemical interaction with the blood without entering the actual blood vessel.

In the second experiment after positioning of a guide catheter the mini-catheter was inserted which was coated with the anti-thrombogenic agent on both its inner surface and its outer surface. It was possible herein to introduce the catheter fully and move it relatively well.

In a third experiment the mini-catheter was inserted after placing of the guiding catheter. The mini-catheter was coated on the inner surface with anti-thrombogenic agent and on the outer surface with an anti-thrombogenic gel. The mini-catheter could now be moved very easily. This indicates a low friction.

When the non-coated mini-catheter and the mini-catheter coated with an anti-thrombogenic agent were tested in an inert liquid such as water or a glycerol dilution in a mix ratio such that the average viscosity of human blood was imitated, no significant differences in friction between the two were observed. Neither of the two became stuck. The very well operating third configuration did not differ in performance from the first and second configurations when they were tested in inert liquid.

The above example demonstrates that coating of a medical device such as a catheter can prevent friction problems in the human or animal body.

## Claims

1. Medical device with reduced frictional properties, **characterized in that** at least two of the contact surfaces inside the device and/or between the device and its environment are provided with means for reducing, changing and/or eliminating biological/biochemical responses of the human or animal body and/or human or animal body fluids which occur in reaction to the contact with the device.

2. Medical device as claimed in claim 1, further **characterized by** means for providing a fluid layer between the contact surfaces inside the device and/or between the device and its environment.

3. Medical device as claimed in claim 1 or 2, **characterized in that** the device comes into contact with blood and the means for reducing, changing and/or eliminating biological/biochemical responses are formed by a surface coating layer in which anti-clotting agents are included.

4. Medical device as claimed in claim 3, **characterized in that** the anti-clotting agents are formed by heparin or derivatives thereof.

5. Medical device as claimed in any of the claims 2-4, **characterized in that** the means for providing a fluid layer are formed by a hydrogel.

6. Medical device as claimed in any of the claims 1-5, **characterized in that** the device is a coaxial catheter system.

7. Medical device as claimed in claim 6, **characterized in that** the outer surfaces of the inner catheter and the inner surfaces of the outer catheter of the coaxial catheter system are provided with means for reducing, changing and/or eliminating biological/biochemical responses and optionally means for providing a fluid layer.

8. Medical device as claimed in claim 6 or 7, **characterized in that** all surfaces of the catheters of the coaxial catheter system are provided with means for reducing, changing and/or eliminating biological/biochemical responses and at least a part of the outer surfaces is also provided with means for providing a fluid layer.

9. Method for reducing the frictional properties of a medical device by providing at least two of the contact surfaces inside the device and/or between the device and its environment with means for reducing, changing and/or eliminating biological/biochemical responses of the human or animal body and/or human or animal body fluids which occur in reaction to the contact with the device.

10. Method as claimed in claim 9, **characterized in that** means are also applied for providing a fluid layer between the contact surfaces inside the device and/or between the device and its environment.
